# EUROPEAN PATENT SPECIFICATION

(11) **EP 3 911 304 B1**
(45) Date of publication and mention of the grant of the patent: **06.09.2023**
(21) Application number: 20700712.1
(22) Date of filing: 14.01.2020
(51) Int. Cl.: A61K 9/20, A61K 9/28, A61K 31/443

(54) **MODIFIED RELEASE TABLET FORMULATIONS CONTAINING PHOSPHODIESTERASE INHIBITORS**
TABLETTENFORMULIERUNGEN MIT MODIFIZIERTER FREISETZUNG, DIE PHOSPHODIESTERASE-INHIBITOREN ENTHALTEN
FORMULATIONS DE COMPRIMÉS À LIBÉRATION MODIFIÉE CONTENANT DES INHIBITEURS DE PHOSPHODIESTÉRASE

(30) Priority: 15.01.2019 EP 19151782
(43) Date of publication of application: 24.11.2021
(73) Proprietor: UNION therapeutics A/S, 2900 Hellerup (DK)
(72) Inventor: RASMUSSEN, Marianne, 2750 Ballerup (DK); HØY, Karin Green, 2750 Ballerup (DK); RAVN, Carsten, 2750 Ballerup (DK); PAJANDER, Jari, 2750 Ballerup (DK); BERTELSEN, Poul E., 2750 Ballerup (DK); PEDERSEN, Gitte Pommergaard, 2750 Ballerup (DK)
(74) Representative: HGF
(86) International application number: PCT/EP2020/050798
(87) International publication number: WO 2020/148271

(56) References cited:
- WO-A1-2011/160632
- US-A1- 2005 186 276
- US-A1- 2007 104 792

## Description

### FIELD OF THE INVENTION

The present invention relates to modified release tablet formulations for oral administration of phosphodiesterase inhibitors. The formulations are useful in the treatment, prevention or alleviation of dermal diseases or conditions.

### BACKGROUND OF THE INVENTION

The drug release and the chemical and physical characteristics of a drug substance, among other factors, can influence the degree of success of obtaining optimal therapy. The use of modified release tablet formulations may control the release of the therapeutic agent and thus control drug absorption from gastrointestinal tract. However, it is often difficult to predict whether a particular modified release formulation will provide the desired release profile, and it has generally been found that it is necessary to carry out considerable experimentation to obtain modified release formulations having the desired effect.

Phosphodiesterases (PDE's) are enzymes that catalyse the hydrolysis of cyclic AMP and/or cyclic GMP in cells to 5-AMP and 5-GMP, respectively, and as such they are critical to cellular regulation of cAMP or cGMP levels. Phosphodiesterase 4 (PDE4), is selective for cAMP. PDE4 is the most important modulator of cAMP expressed in immune and inflammatory cells such as neutrophils, macrophages and T-lymphocytes. As cAMP is a key second messenger in the modulation of inflammatory responses, PDE4 has been found to regulate inflammatory responses of inflammatory cells by modulating proinflammatory cytokines such as TNF-o, IL-2, IFN-γ, GM-CSF and LTB4. Inhibition of PDE4 has therefore become an attractive target for the therapy of inflammatory diseases such as asthma, chronic obstructive pulmonary disease, rheumatoid arthritis, atopic dermatitis, psoriasis, inflammatory bowel disease etc. (M.D. Houslay et al., Drug Discovery Today 10 (22), 2005, pp. 1503-1519). PDE4 inhibitors have been associated with adverse effects found when administered to patients, primarily gastrointestinal side effects such as nausea, diarrhoea, and emesis.

WO2011/169632 discloses the synthesis of the PDE4 inhibitor of formula (I).

In a clinical trial where patients were dosed with immediate release tablets containing the PDE4 inhibitor of formula (I) of the present invention, the results demonstrated that the PDE4 inhibitor was effective in patients with moderate to severe psoriasis vulgaris, but the extent of gastrointestinal related adverse events experienced were unacceptable.

Thus, there exists a need for a pharmaceutical formulation for oral administration of a PDE inhibitor, which could maintain systemic exposure and reduce gastrointestinal adverse events.

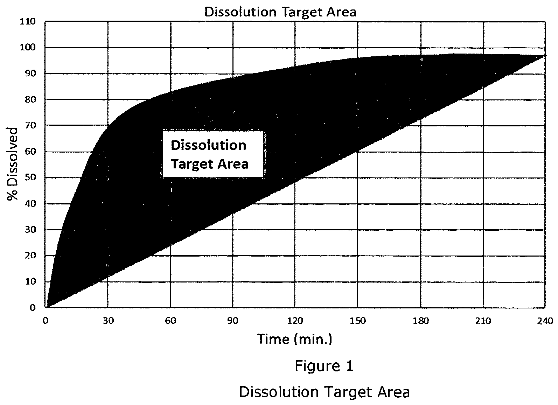

It has been found that beneficial effects with respect to improved tolerability towards gastrointestinal adverse events and maintained systemic exposure have been achieved by formulating a PDE4 inhibitor in a modified release tablet formulation, wherein the in-vitro release is fast in comparison to a typically modified release profile but not yet as fast as for an immediate release tablet where major tolerability issues were seen. The Dissolution target area in Figure 1 illustrates the optimal area.

### SUMMARY OF THE INVENTION

One object of the present invention is to provide modified release tablet formulations according to the claims for oral administration of a PDE inhibitor, which formulations are useful in the treatment, prevention or alleviation of dermal diseases or conditions.

Another object of the present invention is to provide a modified release tablet formulation according to the claims for oral administration of a PDE inhibitor, which shows beneficial effects with respect to maintained systemic exposure and improved tolerability towards reduced gastrointestinal adverse events.

It is hypothesised that an important driver for the gastrointestinal adverse events could be related to the local concentration of the PDE4 inhibitor in the gut and that higher local concentration of the PDE4 inhibitor could cause increased level of gastrointestinal adverse events.

Upon entering the gastrointestinal tract, if the PDE4 inhibitor could slowly be released and dissolved, thus a high local concentration of PDE4 inhibitor could be prevented in the intestinal fluids.

It has been found that when the PDE4 inhibitor was given orally a very narrow absorption window existed in the upper part of the gastrointestinal tract. Therefore, to maintain the systemic exposure the release and dissolution of the drug substance from the modified release tablet formulation must take place in the upper part of the gastrointestinal tract.

In one aspect, the present invention relates to a modified release tablet formulation for oral administration of a PDE inhibitor, comprising:
(i) a PDE inhibitor;
(ii) one or more of a pharmaceutically acceptable hydrophilic matrix former; and
(iii) one or more pharmaceutically acceptable excipients selected from the group consisting of fillers, glidants and lubricants,
wherein:
the hydrophilic matrix former is present in a concentration from about 10 %w/w to about 30 %w/w hydroxypropyl methylcellulose;
the PDE inhibitor is a compound of formula (I): 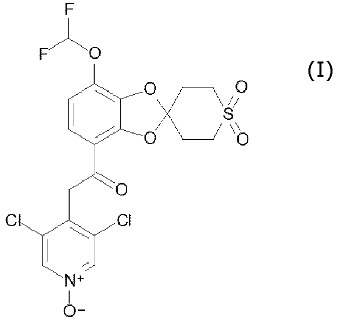
or a pharmaceutically acceptable salt, or polymorphic forms thereof; and the modified release tablet formulation optionally further comprises a pharmaceutically acceptable coating system.

The compound of formula (I), 2-(3,5-Dichloro-1-oxido-pyridine-4-yl)-1-(7-difluoro-methoxy-2',3',5',6-tetrahydro-spiro[1,3-benzodioxole-2, 4'-(4H)-thiopyran-1',1'-dioxide]-4-yl)ethanone, hereafter named Compound A, was disclosed in WO 2011/160632, relating to benzodioxole and benzodioxepine heterocyclic compounds useful as PDE4 inhibitors for use in the treatment, prevention or alleviation of a variety of diseases, such as dermal diseases or conditions, such as proliferative and inflammatory skin disorders, dermatitis, psoriasis, psoriasis vulgaris, atopic dermatitis, seborrheic dermatitis, contact dermatitis, cancer, epidermal inflammation, alopecia, alopecia areata, skin atrophy, steroid induced skin atrophy, skin ageing, photo skin ageing, acne, urticaria, pruritis, and eczema.

The compound A should be understood to include any pharmaceutically acceptable form and salts of the compound. The compound A may be present in a crystalline or amorphous form. Compound A is considered as being a poorly soluble compound. The compound A and salts thereof, and methods for synthesizing the compound, are disclosed in WO 2011/160632, WO 2015/197534, WO 2017/103058, and WO 2018/234299.

In another aspect, the present invention relates to a modified release tablet according to the claims for use in the treatment, prevention or alleviation of dermal diseases or conditions selected from the group consisting of proliferative and inflammatory skin disorders, dermatitis, psoriasis, psoriasis vulgaris (plaque-type psoriasis), atopic dermatitis, seborrheic dermatitis, contact dermatitis, cancer, epidermal inflammation, alopecia, alopecia areata, skin atrophy, steroid induced skin atrophy, skin ageing, photo skin ageing, acne, urticaria, pruritis, and eczema; wherein the modified release tablet is administered orally.

In another aspect, the present invention relates to a modified release tablet formulation according to the claims for use in the oral treatment, prevention or alleviation of psoriasis vulgaris.

In another aspect, the present invention relates to a modified release tablet formulation according to the claims for use in the oral treatment, prevention or alleviation of moderate to severe psoriasis vulgaris.

In another aspect, the present invention relates to a modified release tablet formulation according to the claims wherein the in-vitro release is fast in comparison to a typically modified release profile but not yet as fast as for an immediate release tablet, as indicated in the background section of the present application.

The rate of dissolution will be determined by several factors e.g. the type and quantity of hydrophilic matrix former, excipients (fillers and coating) and the particle size of the drug substance.

The details of one or more embodiments of the inventions are set forth in the description below. Other features, objects and advantages of the inventions will be apparent from the description and claims.

### BRIEF DESCRIPTION OF THE FIGURES

Figure 1. A chart illustrating the dissolution target area; dissolution method: Paddle 75 rpm, 900 ml 0.1N HCl +0.5%SDS, 37°C, HPLC detection.
   The curves presented in figure 2 and 3 describe the release of compound A from the formulations F1 and F2, respectively, when tested by the dissolution method parameters described on page 11.
Figure 2. Dissolution profile for F1.
Figure 3. Dissolution profile for F2.
Figure 4. A chart illustrating the presence of gastrointestinal adverse events in subject, with onset when dosed twice daily by 10mg, 20mg or 30mg of the PDE4 inhibitor of formula (I) of the present invention in Modified Release tablets; each dot represent one gastrointestinal related adverse event and the bar the duration of the adverse event.
Figure 5. A chart illustrating the presence of gastrointestinal adverse events in subject, with onset when dosed twice daily by 10mg, 20mg or 30mg of the PDE4 inhibitor of formula (I) of the present invention in Immediate Release tablets; each dot represent one gastrointestinal related adverse event and the bar the duration of the adverse event.

### DETAILED DISCLOSURE OF THE INVENTION

### Definitions

As used throughout the present specification and appended claims, the following terms have the indicated meaning.

The phrase "phosphodiesterase" as used herein refers to one or more of the phosphodiesterases (PDEs), PDE4, PDE7 and PDE8 being selective for cAMP. PDE4 is the most important modulator of cAMP.

The phrase "PDE inhibitor" as used herein may be any substances which inhibit PDE. The PDE inhibitor is preferably a human PDE inhibitor. The PDE inhibitor is preferably a PDE4 inhibitor. For example, the PDE4 inhibitor could be Compound A, or a pharmaceutically acceptable salt, or polymorphic forms thereof, preferably Compound A, and more preferably the polymorphic form E of Compound A.

The term "treatment" as used herein includes amelioration of a symptom, prevention of an aggravation, maintenance of a remission, prevention of an exacerbation, and prevention of a recurrence. The term "prevention" refers to suppressing occurrence of a symptom.

The term "treatment" may also include the delaying of the progression of the disease, disorder or condition, the amelioration, alleviation or relief of symptoms and complications, and/or cure or elimination of the disease, disorder or condition.

The terms "disease", "disorder" and "condition" as used herein are used interchangeably to specify a state of a patient which is not the normal physiological state of a human being.

The term "hydrophilic matrix former" as used herein includes hydroxypropyl methylcellulose (HPMC). For example, the hydrophilic matrix former is hydroxypropyl methylcellulose, or mixtures thereof.

The term "filler" as used herein includes lactose, for example lactose monohydrate, lactose hydrous or anhydrous, microcrystalline cellulose, mannitol, isomalt, etc. For example, the filler could be lactose monohydrate, microcrystalline cellulose, or a mixture thereof.

The term "filler" as used herein may also function as a binder.

The term "glidant" as used herein includes colloidal silicon dioxide, talc, etc. For example, the glidant could be colloidal silicon dioxide.

The term "lubricant" as used herein includes magnesium stearate, sodium stearyl fumarate, talc, etc. For example, the lubricant could be magnesium stearate.

The term "coating system", as used herein includes HPMC-based coating systems, PVA-based coating systems (polyvinyl alcohol), PVA-PEG based coating systems (polyethylene glycol) or ethylcellulose based functional barrier membrane coating systems.. For example, the coating system could be the PVA-based coating system. The term "about" is used herein to mean approximately, in the region of, roughly, or around.

### Embodiments

References herein to a method of preventing, treating or alleviating a medical condition using the modified release composition are to be interpreted as the modified release composition for use in the prevention, treatment or alleviation of the medical condition.

Embodiments of the modified release tablet formulation may include one or more of the following features.

In one embodiment of the modified release tablet formulation the PDE inhibitor is a PDE4 inhibitor.

In another embodiment the PDE4 inhibitor is evenly distributed.

In another embodiment the PDE4 inhibitor is micronized.

In another embodiment the PDE4 inhibitor is crystalline and micronized.

The PDE4 inhibitor is compound A.

In another embodiment the PDE4 inhibitor is preferably the polymorphic form E of Compound A.

In another embodiment compound A is micronized. In another embodiment the polymorphic Form E of compound A is micronized.

In another embodiment compound A is evenly distributed. In another embodiment the polymorphic Form E of compound A is evenly distributed.

In another embodiment, compound A is crystalline and micronized.

In another embodiment, the polymorphic Form E of compound A is crystalline and micronized.

In another embodiment the modified release tablet formulation contains a hydrophilic matrix former, or mixtures thereof. For example, the hydrophilic matrix former is hydroxypropyl methylcellulose (HPMC), or mixtures thereof.

The hydrophilic matrix former is present at various concentrations and combinations from about 10 %w/w to about 30 %w/w HPMC, for example from about 15 %w/w to about 25 %w/w HPMC, and specifically 17. 5 %w/w HMPC.

In another embodiment the modified release tablet formulation could comprise one or more fillers/binders selected from lactose monohydrate, lactose hydrous or anhydrous, microcrystalline cellulose, and mixtures thereof. For example, the filler could be lactose monohydrate.

The filler could be present at various concentrations from about 30 %w/w to about 78 %w/w of lactose monohydrate and from about 0 to about 40 %w/w of microcrystalline cellulose. For example, the filler could be lactose monohydrate in a 71 %w/w.

In another embodiment the modified release tablet formulation could comprise one or more glidants. For example, the glidant could be colloidal silicon dioxide.

The glidant could be present at various concentrations from about 0.1 %w/w to about 2 %w/w of colloidal silicon dioxide, for example from about 0.2 %w/w to about 1 %w/w, and specifically 0.5%w/w.

In another embodiment the modified release tablet formulation could comprise one or more lubricants. For example, the lubricant could be magnesium stearate.

The lubricant could be present at various concentrations from about 0.1 %w/w to about 2 %w/w of magnesium stearate, for example from about 0.5 %w/w to about 1.5%w/w, and specifically 1.0 %w/w.

In another embodiment the modified release tablet formulation could comprise a film coating of the tablet cores.

In another embodiment the coating system could be a PVA-based coating system. For example, the coating system could be Opadry ^{®} II. For example the coating system could be present in an amount from about 3% to about 5 % weight gain of the tablet formulation, and specifically a 4 % weight gain.

In another embodiment of the present invention, the particle size distribution of the PDE inhibitor could have a D₅₀ ≤ 25 µm, for example D₅₀ ≤ 20 µm, D₅₀ ≤ 10 µm, D₅₀ ≤ 5 um, or D₅₀ ≤ 3 µm.

In another aspect, the amount of the PDE inhibitor may range from about 5 mg to about 60 mg. The amount of PDE inhibitor may for example range from 10 mg to 50 mg, from 20 mg to 45 mg, and from 30 mg to 40 mg.

Also disclosed but not claimed is a method of treating psoriasis vulgaris. The method includes administering to a patient in need thereof, a modified release tablet formulation containing a PDE inhibitor.

Also disclosed but not claimed is a method of treating psoriasis vulgaris in a patient in need thereof, which method includes administering a modified release tablet formulation containing compound A in a concentration of 10 mg.

Also disclosed but not claimed is a method of treating psoriasis vulgaris in a patient in need thereof, which method includes administering a modified release tablet formulation containing compound A in a concentration of 30 mg.

Also disclosed but not claimed is a method of treating psoriasis vulgaris in a patient in need thereof, which method includes administering a modified release tablet formulation containing compound A in a concentration of 30 mg, twice daily.

Also disclosed is a process for the preparation of the modified release tablet formulation wherein the manufacturing process consisted of blending and sieving steps of the drug substance and excipients followed by direct compression, or roller compaction followed by compression, and finally optionally coating.

### EXAMPLES

### EXAMPLE 1

### Modified release tablet formulations

**Table 1. Modified release tablet formulations (F) for 10 mg and 30 mg dose strengths (in percentage w/w).**

| Ingredient | F 1 (10mg) | F2 (30mg) | F 3 (30mg) | F 4 (30mg) | F 5 (30mg) | F 6 (30mg) | F7 (10mg) |
|---|---|---|---|---|---|---|---|
| | Direct compression | Direct compression | Direct compression | Direct compression | Direct compression | Roller compac tion | Roller compac tion |
| **Drug substance** | | | | | | | |
| Compound A (micronized) | 3.3 % | 10.0% | 10.0% | 10.0% | 10.0% | 10.0% | 3.3% |
| **Excipients** | | | | | | | |
| Lactose monohydrate | 77.7 % | 71.0 % | 73.5 % | 36.7 % | 50.0 % | 49.5 % | 49.5 % |
| Microcrystallin e cellulose | - | - | - | 36.7 % | 14.0 % | 14.0 % | 20.5 % |
| Hydroxypropyl methylcellulose (Methocel^{®} E50 LV | - | - | - | - | 25.0 % | 25.0 % | 20.0 % |
| Hydroxypropyl methylcellulose (Methocel^{®} K100 LV) | 17.5 % | 17.5 % | 15.0 % | 15.0 % | - | - | 5.0% |
| Colloidal silicon dioxide | 0.5 % | 0.5 % | 0.5 % | 0.5 % | - | 0.5 % | 0.5 % |
| Magnesium Stearate | 1.0 % | 1.0% | 1.0% | 1.0% | 1.0% | 1.0% | 1.0% |
| **Film coating** | | | | | | | |
| Coating system (% weight gain) | 4% Opadry ^{®} II | 4% Opadry ^{®} II | Not coated | 4% Opadry ^{®}II | Not coated | 4 % Surelea se/Opa dry | Not coated |

### EXAMPLE 2

### Dissolution profiles for formulations F1 and F2

The release of compound A from the modified release tablet was investigated by in-vitro dissolution method, see below.

### Dissolution method:

Dissolution apparatus: USP/Ph.Eur. app.II (paddles)
75 rpm, 900 ml 0.1N HCl +0.5%SDS, 37°C, HPLC detection.
Modified release formulation F1
Dissolution: % of declared amount

| time (min) | mean | SD |
|---|---|---|
| 6 | 7 | 1.0 |
| 12 | 15 | 1.1 |
| 20 | 22 | 1.3 |
| 30 | 28 | 1.7 |
| 45 | 36 | 2.2 |
| 60 | 42 | 2.2 |
| 90 | 54 | 2.6 |
| 120 | 64 | 3.0 |
| 180 | 81 | 2.6 |

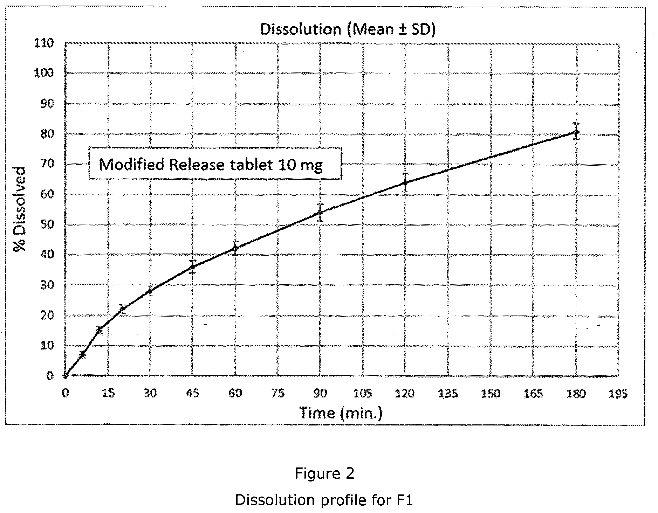

Figure 2 shows the dissolution profile for F1
Modified release formulation F2
Dissolution: % of declared amount

| time (min) | mean | SD |
|---|---|---|
| 6 | 7 | 1.0 |
| 12 | 14 | 0.7 |
| 20 | 23 | 1.0 |
| 30 | 31 | 1.7 |
| 45 | 39 | 2.7 |
| 60 | 46 | 3.6 |
| 90 | 56 | 4.3 |
| 120 | 66 | 4.1 |
| 180 | 82 | 5.6 |

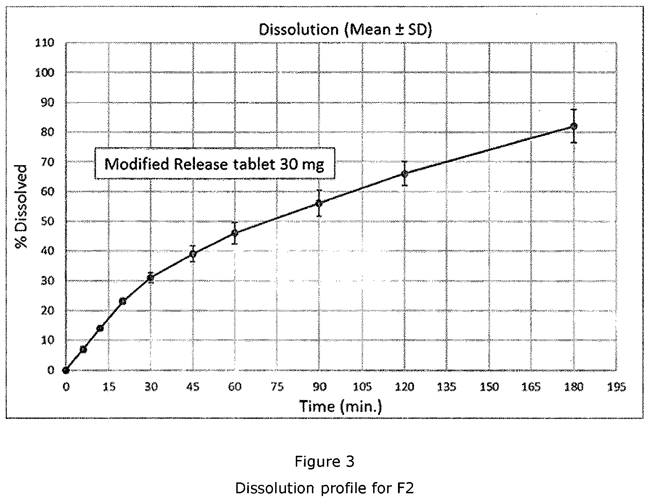

Figure 3 shows the dissolution profile for F2

### EXAMPLE 3

In an oral dose clinical trial, healthy subjects were dosed with the PDE4 inhibitor of formula (I) in the Modified Release tablet formulation of the present invention. The subjects received twice daily doses of: 10 mg of the compound of formula (I) on Days 1-2, 20 mg of the compound of formula (I) on Days 3-4, 30 mg of the compound of formula (I) on Days 5-6, 40 mg of the compound of formula (I) on Days 7-8, 50 mg of the compound of formula (I) on Days 9-10, and 60 mg of the compound of formula (I) on Days 11-17.

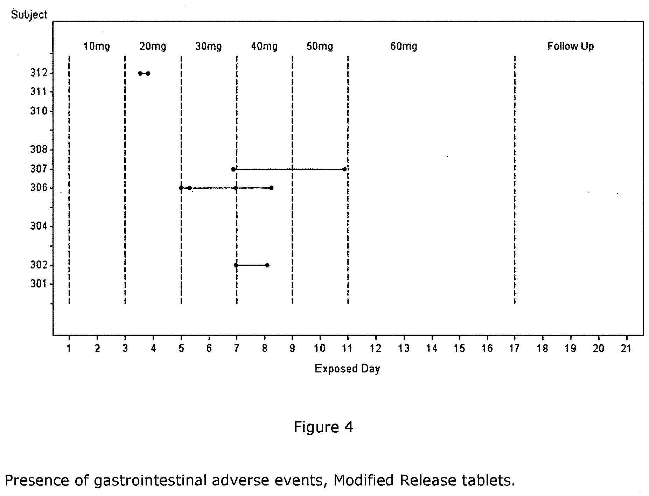

Gastrointestinal adverse events in the subjects were collected in connection with onset when dosed by 10mg, 20mg or 30mg of the PDE4 inhibitor of formula (I), as shown in Figure 4.

Results from this Modified Release tablet formulation study (Figure 4) was compared with the results from a previous completed comparable oral dose clinical trial, where healthy subjects were dosed with the PDE4 inhibitor of formula (I) of the present invention in an Immediate Release tablet formulation.

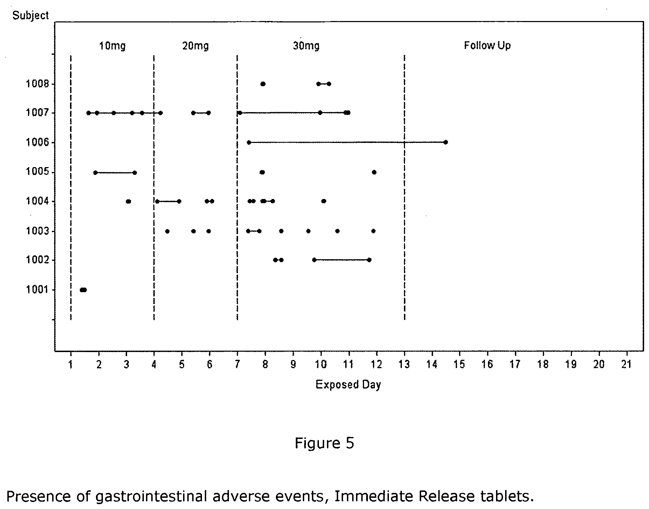

In the Immediate Release tablet formulation study, subjects received twice daily doses of: 10 mg of the PDE4 inhibitor of formula (I) of the present invention on Days 1-3, 20 mg of the compound of formula (I) on Days 4-6, and 30 mg of the compound of formula (I) on Days 7-13. Gastrointestinal adverse events in the subjects were collected in connection with onset when dosed by 10mg, 20mg or 30mg of the PDE4 inhibitor of formula (I). The results which are shown in Figure 5, demonstrates that the Immediate Release tablet formulation was poorly tolerated.

When comparing the results shown in Figure 4 (presence of gastrointestinal related adverse events in subjects when dosed with Modified Release tablets containing the PDE4 inhibitor of formula (I)) with the results shown in Figure 5 (presence of gastrointestinal related adverse events in subjects when dosed with Immediate Release tablets containing the PDE4 inhibitor of formula (I)), the Modified Release tablet formulation study shows that the total number of gastrointestinal adverse events was clinically significantly lower when compared to the immediate release tablets.

## Claims

1. A modified release tablet formulation comprising:
(i) a PDE inhibitor;
(ii) one or more of a pharmaceutically acceptable hydrophilic matrix former; and
(iii) one or more pharmaceutically acceptable excipients selected from the group consisting of fillers, glidants and lubricants,
wherein:
the hydrophilic matrix former is present in a concentration from about 10 %w/w to about 30 %w/w hydroxypropyl methylcellulose;
the PDE inhibitor is a compound of formula (I): 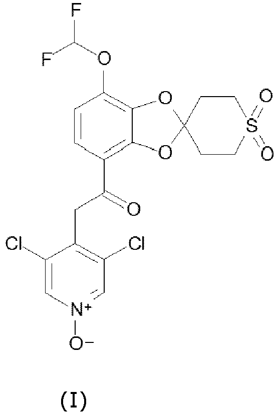
or a pharmaceutically acceptable salt, or polymorphic forms thereof; and
the modified release tablet formulation optionally further comprises a pharmaceutically acceptable coating system.

2. The modified release tablet formulation according to claim 1, wherein the hydrophilic matrix former comprises one or more of hydroxypropyl methylcellulose, or mixtures thereof.

3. The modified release tablet formulation according to claim 2, wherein the hydroxypropyl methylcellulose is hypromellose 2910, hypromellose 2208, or mixtures thereof.

4. The modified release tablet formulation according to any one of claims 1 to 3, wherein the hydrophilic matrix former is present in a concentration from about 15 %w/w to about 25 %w/w hydroxypropyl methylcellulose.

5. The modified release tablet formulation according to claim 4, wherein the hydrophilic matrix former is present in a concentration of 17,5 %w/w hydroxypropyl methylcellulose.

6. The modified release tablet formulation according to any one of claims 1 to 5, wherein one of the pharmaceutically acceptable excipients is a filler, selected from lactose monohydrate and microcrystalline cellulose, or a mixture thereof.

7. The modified release tablet formulation according to claim 6, wherein the filler is present in a concentration from about 30 % to about 78% w/w of lactose monohydrate and from 0 to about 40 %w/w of microcrystalline cellulose.

8. The modified release tablet formulation according to claim 7, wherein the filler is present in a concentration of about 71 %w/w lactose monohydrate.

9. The modified release tablet formulation according to any one of claims 1 to 7, wherein one of the pharmaceutically acceptable excipients is a glidant, which is colloidal silicon dioxide;
optionally wherein the glidant is present in a concentration from about 0.1 %w/w to about 2 %w/w of colloidal silicon dioxide, for example from about 0.2 %w/w to about 1 %w/w, and specifically 0.5 %w/w.

10. The modified release tablet formulation according to any one of claims 1 to 9, wherein one of the pharmaceutically acceptable excipients is a lubricant, which is magnesium stearate;
optionally wherein the lubricant is present in a concentration from about 0.1 %w/w to about 2 %w/w of magnesium stearate, for example from about 0.5 %w/w to about 1.5 %w/w, and specifically 1.0 %w/w.

11. The modified release tablet formulation according to any one of claims 1 to 10, wherein the coating system comprises a coating system selected from: a hydroxypropyl methylcellulose-based coating system, a polyvinyl alcohol-based coating system, a polyvinyl alcohol-polyethylene glycol-based coating system and an ethylcellulose based functional barrier membrane coating system.

12. The modified release tablet formulation according to any one of claims 1 to 11, wherein the modified release tablet formulation comprises a coating system.

13. The modified release tablet formulation according to claim 12, wherein the coating is present in an amount from about 3% to about 5% weight gain of the tablet formulation.

14. The modified release tablet formulation according to any one of claims 1 to 12, wherein the compound of formula (I) is in micronized form.

15. The modified release tablet formulation according to claim 1, wherein the formulation consists of about 3.3 %w/w micronized 2-(3,5-dichloro-1-oxido-pyridine-4-yl)-1-(7-difluoromethoxy-2',3',5',6'-tetrahydro-spiro[1,3-benzodioxole-2, 4'-(4H)-thiopyran-1',1'-dioxide]-4-yl)ethanone, about 17.5 %w/w hypromellose, about 77.7 %w/w lactose monohydrate, about 0.5 %w/w colloidal silicon dioxide, about 1.0 %w/w magnesium stearate; and optionally a PVA-based coating system.

16. The modified release tablet formulation according claim 1, wherein the formulation consists of about 10.0 %w/w micronized 2-(3,5-dichloro-1-oxido-pyridine-4-yl)-1-(7-difluoromethoxy-2',3',5',6'-tetrahydro-spiro[1,3-benzodioxole-2, 4'-(4H)-thiopyran-1',1'-dioxide]-4-yl)ethanone, about 17.5 %w/w hypromellose, about 71.0 %w/w lactose monohydrate, about 0.5 %w/w colloidal silicon dioxide, about 1.0 %w/w magnesium stearate; and optionally a PVA-based coating system.

17. The modified release tablet formulation according to any one of claims 1 to 16, wherein the tablet is coated with a PVA-based coating system;
optionally wherein the coating is present in an amount of 4% weight gain of the tablet formulation.

18. The modified release tablet formulation according to any one of claims 1 to 17, wherein the compound of formula (I) has a particle size distribution with D₅₀ ≤ 5 µm.

19. The modified release tablet formulation according to any one of claims 1 to 18, wherein the compound of formula (I) is 2-(3,5-dichloro-1-oxido-pyridine-4-yl)-1-(7-difluoromethoxy-2',3',5',6'-tetrahydro-spiro[1,3-benzodioxole-2, 4'-(4H)-thiopyran-1',1'-dioxide]-4-yl)ethanone, polymorphic form E.

20. The modified release tablet formulation according to any one of claims 1 to 19, wherein the PDE inhibitor is present in an amount from about 5 mg to about 60 mg;
optionally wherein:
(i) the PDE inhibitor is present in an amount of 10 mg;
(ii) the PDE inhibitor is present in an amount of 20 mg;
(iii) the PDE inhibitor is present in an amount of 30 mg; or
(iv) the PDE inhibitor is present in an amount of 40 mg.

21. The modified release tablet formulation according to any one of claims 1 to 20, for use in the treatment, prevention or alleviation of a dermal disease or condition selected from the group consisting of proliferative and inflammatory skin disorders, dermatitis, psoriasis, psoriasis vulgaris (plaque-type psoriasis), atopic dermatitis, seborrheic dermatitis, contact dermatitis, cancer, epidermal inflammation, alopecia, alopecia areata, skin atrophy, steroid induced skin atrophy, skin ageing, photo skin ageing, acne, urticaria, pruritis, and eczema;
wherein the modified release tablet formulation is administered orally.

22. The modified release tablet formulation, according to any one of claims 1 to 20, for use in the treatment of an inflammatory skin disorder, wherein the modified release tablet formulation is administered orally.

23. The modified release tablet formulation according to any one of claims 1 to 20, for use in the treatment of psoriasis, wherein the modified release tablet formulation is administered orally;
optionally wherein:
(i) the psoriasis is psoriasis vulgaris; or
(ii) moderate to severe psoriasis vulgaris.

24. The modified release tablet formulation according to any one of claims 1 to 20, for use in the treatment of atopic dermatitis, wherein the modified release tablet formulation is administered orally.

## Patentansprüche

1. Tablettenformulierung mit modifizierter Freisetzung, umfassend:
(i) einen PDE-Hemmer;
(ii) eines oder mehrere von einem pharmazeutisch unbedenklichen hydrophilen Matrixbildner; und
(iii) einen oder mehrere pharmazeutisch unbedenklich Hilfsstoffe, die aus der Gruppe ausgewählt sind, die aus Füllstoffen, Fließregulierungsmitteln und Schmiermitteln besteht,
wobei:
der hydrophile Matrixbildner in einer Konzentration von etwa 10 Gew.-% bis etwa 30 Gew.-% Hydroxypropylmethylcellulose vorliegt;
der PDE-Hemmer eine Verbindung der Formel (I) ist: 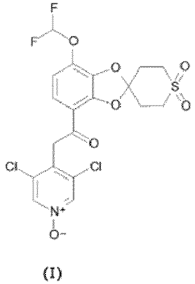
oder ein pharmazeutisch unbedenkliches Salz oder polymorphe Formen davon; und
die Tablettenformulierung mit modifizierter Freisetzung ferner optional ein pharmazeutisch unbedenkliches Beschichtungssystem umfasst.

2. Tablettenformulierung mit modifizierter Freisetzung nach Anspruch 1, wobei der hydrophile Matrixbildner eines oder mehrere von Hydroxypropylmethylcellulose oder Mischungen davon umfasst.

3. Tablettenformulierung mit modifizierter Freisetzung nach Anspruch 2, wobei die Hydroxypropylmethylcellulose Hypromellose 2910, Hypromellose 2208 oder Mischungen davon ist.

4. Tablettenformulierung mit modifizierter Freisetzung nach einem der Ansprüche 1 bis 3, wobei der hydrophile Matrixbildner in einer Konzentration von etwa 15 Gew.-% bis etwa 25 Gew.-% Hydroxypropylmethylcellulose vorliegt.

5. Tablettenformulierung mit modifizierter Freisetzung nach Anspruch 4, wobei der hydrophile Matrixbildner in einer Konzentration von 17,5 Gew.-% Hydroxypropylmethylcellulose vorliegt.

6. Tablettenformulierung mit modifizierter Freisetzung nach einem der Ansprüche 1 bis 5, wobei einer der pharmazeutisch unbedenklichen Hilfsstoffe ein Füllstoff ist, der aus Lactose-Monohydrat und mikrokristalliner Cellulose oder einer Mischung davon ausgewählt ist.

7. Tablettenformulierung mit modifizierter Freisetzung nach Anspruch 6, wobei der Füllstoff in einer Konzentration von etwa 30 Gew.-% bis etwa 78 Gew.-% Lactose-Monohydrat und von 0 bis etwa 40 Gew.-% mikrokristalline Cellulose vorliegt.

8. Tablettenformulierung mit modifizierter Freisetzung nach Anspruch 7, wobei der Füllstoff in einer Konzentration von etwa 71 Gew.-% Lactose-Monohydrat vorliegt.

9. Tablettenformulierung mit modifizierter Freisetzung nach einem der Ansprüche 1 bis 7, wobei einer der pharmazeutisch unbedenklichen Hilfsstoffe ein Fließregulierungsmittel ist, das kolloidales Siliciumdioxid ist;
optional wobei das Fließregulierungsmittel in einer Konzentration von etwa 0,1 Gew.-% bis etwa 2 Gew.-% kolloidales Siliciumdioxid vorliegt, beispielsweise von etwa 0,2 Gew.-% bis etwa 1 Gew.-% und insbesondere 0,5 Gew.-%.

10. Tablettenformulierung mit modifizierter Freisetzung nach einem der Ansprüche 1 bis 9, wobei einer der pharmazeutisch unbedenklichen Hilfsstoffe ein Schmiermittel ist, das Magnesiumstearat ist;
optional wobei das Schmiermittel in einer Konzentration von etwa 0,1 Gew.-% bis etwa 2 Gew.-% Magnesiumstearat vorliegt, beispielsweise von etwa 0,5 Gew.-% bis etwa 1,5 Gew.-% und insbesondere 1,0 Gew.-%.

11. Tablettenformulierung mit modifizierter Freisetzung nach einem der Ansprüche 1 bis 10, wobei das Beschichtungssystem ein Beschichtungssystem umfasst, das aus Folgendem ausgewählt ist: einem Beschichtungssystem auf Hydroxypropylmethylcellulose-Basis, einem Beschichtungssystem auf Polyvinylalkohol-Basis, einem Beschichtungssystem auf Polyvinylalkohol-Polyethylenglykol-Basis und einem auf Ethylcellulose basierenden funktionellen Sperrmembran-Beschichtungssystem.

12. Tablettenformulierung mit modifizierter Freisetzung nach einem der Ansprüche 1 bis 11, wobei die Tablettenformulierung mit modifizierter Freisetzung ein Beschichtungssystem umfasst.

13. Tablettenformulierung mit modifizierter Freisetzung nach Anspruch 12, wobei die Beschichtung in einer Menge eines Gewichtszuwachses von etwa 3 % bis etwa 5 % der Tablettenformulierung vorliegt.

14. Tablettenformulierung mit modifizierter Freisetzung nach einem der Ansprüche 1 bis 12, wobei die Verbindung der Formel (I) in mikronisierter Form vorliegt.

15. Tablettenformulierung mit modifizierter Freisetzung nach Anspruch 1, wobei die Formulierung aus etwa 3,3 Gew.-% mikronisiertem 2-(3,5-Dichlor-1-oxido-pyridin-4-yl)-1-(7-diflluormethoxy-2',3',5',6'-tetrahydro-spiro[1,3-benzodioxol-2, 4'-(4H)-thiopyran-1',1'-dioxid]-4-yl)ethanon, etwa 17,5 Gew.-% Hypromellose, etwa 77,7 Gew.-% Lactose-Monohydrat, etwa 0,5 Gew.-% kolloidalem Siliciumdioxid, etwa 1,0 Gew.-% Magnesiumstearat; und optional einem PVA-basierten Beschichtungssystem besteht.

16. Tablettenformulierung mit modifizierter Freisetzung nach Anspruch 1, wobei die Formulierung aus etwa 10,0 Gew.-% mikronisiertem 2-(3,5-Dichlor-1-oxido-pyridin-4-yl)-1-(7-difluormethoxy-2',3',5',6'-tetrahydro-spiro[1,3-benzodioxol-2,4'-(4H)-thiopyran-1',1'-dioxid]-4-yl)ethanon, etwa 17,5 Gew.-% Hypromellose, etwa 71,0 Gew.-% Lactose-Monohydrat, etwa 0,5 Gew.-% kolloidalem Siliciumdioxid, etwa 1,0 Gew.-% Magnesiumstearat; und optional einem PVA-basiertes Beschichtungssystem besteht.

17. Tablettenformulierung mit modifizierter Freisetzung nach einem der Ansprüche 1 bis 16, wobei die Tablette mit einem PVA-basierten Beschichtungssystem beschichtet ist;
optional wobei die Beschichtung in einer Menge eines Gewichtszuwachses von 4 % der Tablettenformulierung vorliegt.

18. Tablettenformulierung mit modifizierter Freisetzung nach einem der Ansprüche 1 bis 17, wobei die Verbindung der Formel (I) eine Partikelgrößenverteilung mit D₅₀ ≤ 5 µm aufweist.

19. Tablettenformulierung mit modifizierter Freisetzung nach einem der Ansprüche 1 bis 18, wobei die Verbindung der Formel (I) 2-(3,5-Dichlor-1-oxido-pyridin-4-yl)-1-(7-difluormethoxy-2',3',5',6'-tetrahydro-spiro[1,3-benzodioxol-2,4'-(4H)-thiopyran-1',1'-dioxid]-4-yl)ethanon, polymorphe Form E ist.

20. Tablettenformulierung mit modifizierter Freisetzung nach einem der Ansprüche 1 bis 19, wobei der PDE-Hemmer in einer Menge von etwa 5 mg bis etwa 60 mg vorliegt;
wobei optional:
(i) der PDE-Hemmer in einer Menge von 10 mg vorliegt;
(ii) der PDE-Hemmer in einer Menge von 20 mg vorliegt;
(iii) der PDE-Hemmer in einer Menge von 30 mg vorliegt; oder
(iv) der PDE-Hemmer in einer Menge von 40 mg vorliegt.

21. Tablettenformulierung mit modifizierter Freisetzung nach einem der Ansprüche 1 bis 20 zur Verwendung bei der Behandlung, Vorbeugung oder Linderung einer Hauterkrankung oder eines Hautzustands, die/der aus der Gruppe ausgewählt ist, die aus proliferativen und entzündlichen Hauterkrankungen, Dermatitis, Psoriasis, Psoriasis vulgaris (Plaque-Typ-Psoriasis), atopischer Dermatitis, seborrhoischer Dermatitis, Kontaktdermatitis, Krebs, epidermaler Entzündung, Alopezie, Alopecia areata, Hautatrophie, steroidinduzierter Hautatrophie, Hautalterung, lichtbedingter Hautalterung, Akne, Urtikaria, Pruritis und Ekzem besteht,
wobei die Tablettenformulierung mit modifizierter Freisetzung oral verabreicht wird.

22. Tablettenformulierung mit modifizierter Freisetzung nach einem der Ansprüche 1 bis 20 zur Verwendung bei der Behandlung einer entzündlichen Hauterkrankung, wobei die Tablettenformulierung mit modifizierter Freisetzung oral verabreicht wird.

23. Tablettenformulierung mit modifizierter Freisetzung nach einem der Ansprüche 1 bis 20 zur Verwendung bei der Behandlung von Psoriasis, wobei die Tablettenformulierung mit modifizierter Freisetzung oral verabreicht wird;
wobei optional:
(i) die Psoriasis Psoriasis vulgaris ist; oder
(ii) mittelschwere bis schwere Psoriasis vulgaris.

24. Tablettenformulierung mit modifizierter Freisetzung nach einem der Ansprüche 1 bis 20 zur Verwendung bei der Behandlung von atopischer Dermatitis, wobei die Tablettenformulierung mit modifizierter Freisetzung oral verabreicht wird.

## Revendications

1. Formulation de comprimé à libération modifiée comprenant :
(i) un inhibiteur de la PDE ;
(ii) un ou plusieurs agents hydrophiles de formage de matrice pharmaceutiquement acceptables ; et
(iii) un ou plusieurs excipients pharmaceutiquement acceptables choisis parmi le groupe composé d'agents de charge, d'agents glissants et de lubrifiants,
dans laquelle :
l'agent hydrophile de formage de matrice est présent à une concentration d'environ 10 % p/p à environ 30 % p/p d'hydroxypropylméthylcellulose ;
l'inhibiteur de la PDE est un composé de formule (I) : 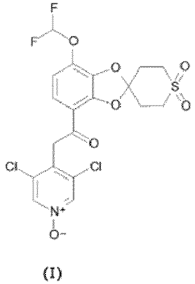
ou un sel pharmaceutiquement acceptable ou formes polymorphiques de celle-ci ; et
la formulation du comprimé à libération modifiée comprend en outre éventuellement un système d'enrobage pharmaceutiquement acceptable.

2. Formulation de comprimé à libération modifiée selon la revendication 1, dans laquelle l'agent de formage de matrice hydrophile comprend une ou plusieurs parmi hydroxypropylméthylcellulose, ou mélanges de celles-ci.

3. Formulation de comprimé à libération modifiée selon la revendication 2, dans laquelle l'hydroxypropylméthylcellulose est l'hypromellose 2910, l'hypromellose 2208 ou des mélanges de celles-ci.

4. Formulation de comprimé à libération modifiée selon l'une quelconque des revendications 1 à 3, dans laquelle l'agent hydrophile de formage de matrice est présent à une concentration d'environ 15 % p/p à environ 25 % p/p d'hydroxypropylméthylcellulose.

5. Formulation de comprimé à libération modifiée selon la revendication 4, dans laquelle l'agent hydrophile de formage de matrice est présent à une concentration d'hydroxypropylméthylcellulose de 17,5 % p/p.

6. Formulation de comprimé à libération modifiée selon l'une quelconque des revendications 1 à 5, dans laquelle l'un des excipients pharmaceutiquement acceptables est un agent de charge, choisi parmi le lactose monohydraté et la cellulose microcristalline, ou un mélange de ceux-ci.

7. Formulation de comprimé à libération modifiée selon la revendication 6, dans laquelle l'agent de charge est présent à une concentration d'environ 30 % à environ 78 % p/p de lactose monohydraté et de 0 à environ 40 % p/p de cellulose microcristalline.

8. Formulation de comprimé à libération modifiée selon la revendication 7, dans laquelle l'agent de charge est présent à une concentration d'environ 71 % p/p de lactose monohydraté.

9. Formulation de comprimé à libération modifiée selon l'une quelconque des revendications 1 à 7, dans laquelle l'un des excipients pharmaceutiquement acceptables est un agent glissant, qui est le dioxyde de silicium colloïdal ;
éventuellement dans laquelle l'agent glissant est présent à une concentration d'environ 0,1 % p/p à environ 2 % p/p de dioxyde de silicium colloïdal, par exemple d'environ 0,2 % p/p à environ 1 % p/p, et spécifiquement 0,5 % p/p.

10. Formulation de comprimé à libération modifiée selon l'une quelconque des revendications 1 à 9, dans laquelle l'un des excipients pharmaceutiquement acceptables est un lubrifiant, qui est le stéarate de magnésium ;
éventuellement dans laquelle le lubrifiant est présent à une concentration d'environ 0,1 % p/p à environ 2 % p/p de stéarate de magnésium, par exemple d'environ 0,5 % p/p à environ 1,5 % p/p, et spécifiquement 1,0 % p/p.

11. Formulation de comprimé à libération modifiée selon l'une quelconque des revendications 1 à 10, dans laquelle le système d'enrobage comprend un système d'enrobage choisi parmi : un système d'enrobage à base d'hydroxypropylméthylcellulose, un système d'enrobage à base d'alcool polyvinylique, un système d'enrobage à base d'alcool polyvinylique-polyéthylène glycol et un système d'enrobage à membrane barrière fonctionnelle à base d'éthylcellulose.

12. Formulation de comprimé à libération modifiée selon l'une quelconque des revendications 1 à 11, la formulation du comprimé à libération modifiée comprenant un système d'enrobage.

13. Formulation de comprimé à libération modifiée selon la revendication 12, dans laquelle l'enrobage est présent en une quantité d'environ 3 % à environ 5 % de gain de poids de la formulation de comprimé.

14. Formulation de comprimé à libération modifiée selon l'une quelconque des revendications 1 à 12, dans laquelle le composé de formule (I) est sous forme micronisée.

15. Formulation de comprimé à libération modifiée selon la revendication 1, la formulation consistant en environ 3,3 % p/p de 2-(3,5-dichloro-1-oxydo-pyridine-4-yl)-1-(7-difluorométhoxy-2',3',5',6'-tétrahydro-spiro[1,3-benzodioxole-2, 4'-(4H)-thiopyran-1',1'-dioxyde]-4-yl)éthanone micronisée, environ 17,5 % p/p d'hypromellose, environ 77,7% p/p de lactose monohydraté, environ 0,5 % p/p de dioxyde de silicium colloïdal, environ 1,0 % p/p de stéarate de magnésium ; et éventuellement un système d'enrobage à base de PVA.

16. Formulation de comprimé à libération modifiée selon la revendication 1, la formulation consistant en environ 10,0 % p/p de 2-(3,5-dichloro-1-oxydo-pyridine-4-yl)-1-(7-difluorométhoxy-2',3',5',6'-tétrahydro-spiro[1,3-benzodioxole-2, 4'-(4H)-thiopyran-1',1'-dioxyde]-4-yl)éthanone micronisée, environ 17,5 % p/p d'hypromellose, environ 71,0 % p/p de lactose monohydraté, environ 0,5 % p/p de dioxyde silicium colloïdal, environ 1,0 % p/p de stéarate de magnésium ; et éventuellement un système d'enrobage à base de PVA.

17. Formulation de comprimé à libération modifiée selon l'une quelconque des revendications 1 à 16, le comprimé étant enrobé avec un système d'enrobage à base de PVA ;
éventuellement, l'enrobage étant présent en une quantité de 4 % de gain de poids de la formulation de comprimé.

18. Formulation de comprimé à libération modifiée selon l'une quelconque des revendications 1 à 17, dans laquelle le composé de formule (I) comporte une distribution granulométrique avec un D₅₀ ≤ 5 µm.

19. Formulation de comprimé à libération modifiée selon l'une quelconque des revendications 1 à 18, dans laquelle le composé de formule (I) est la 2-(3,5-dichloro-1-oxydo-pyridine-4-yl)-1-(7-difluorométhoxy-2',3',5',6'-tétrahydro-spiro[1,3-benzodioxole-2, 4'-(4H)-thiopyran-1',1'-dioxyde]-4-yl)éthanone, forme polymorphique E.

20. Formulation de comprimé à libération modifiée selon l'une quelconque des revendications 1 à 19, dans laquelle l'inhibiteur de la PDE est présent en une quantité d'environ 5 mg à environ 60 mg ;
éventuellement dans laquelle :
(i) l'inhibiteur de la PDE est présent en une quantité de 10 mg ;
(ii) l'inhibiteur de la PDE est présent en une quantité de 20 mg ;
(iii) l'inhibiteur de la PDE est présent en une quantité de 30 mg ; ou
(iv) l'inhibiteur de la PDE est présent en une quantité de 40 mg.

21. Formulation de comprimé à libération modifiée selon l'une quelconque des revendications 1 à 20, pour l'utilisation dans le traitement, la prévention ou l'atténuation d'une maladie ou d'une affection cutanée choisie parmi le groupe constitué des troubles cutanés prolifératifs et inflammatoires, dermatite, psoriasis, psoriasis vulgaris (psoriasis en plaques), dermatite atopique, dermatite séborrhéique, dermatite de contact, cancer, inflammation de l'épiderme, alopécie, alopécie en aires, atrophie cutanée, atrophie cutanée induite par les stéroïdes, vieillissement cutané, photo-vieillissement cutané, acné, urticaire, prurit et eczéma ;
la formulation de comprimé à libération modifiée étant administrée par voie orale.

22. Formulation de comprimé à libération modifiée selon l'une quelconque des revendications 1 à 20, pour l'utilisation dans le traitement d'un trouble inflammatoire de la peau, la formulation de comprimé à libération modifiée étant administrée par voie orale.

23. Formulation de comprimé à libération modifiée selon l'une quelconque des revendications 1 à 20, pour l'utilisation dans le traitement du psoriasis, la formulation de comprimé à libération modifiée étant administrée par voie orale ;
éventuellement :
(i) le psoriasis étant le psoriasis vulgaris ; ou
(ii) le psoriasis vulgaris modéré à sévère.

24. Formulation de comprimé à libération modifiée selon l'une quelconque des revendications 1 à 20, pour l'utilisation dans le traitement de la dermatite atopique, la formulation de comprimé à libération modifiée étant administrée par voie orale.
